# EUROPEAN PATENT APPLICATION

(11) **EP 2 893 917 A1**
(43) Date of publication of application: **15.07.2015**
(21) Application number: 15275005.5
(22) Date of filing: 09.01.2015
(51) Int. Cl.: A61K 6/087, A61C 13/00

(54) **Prosthodontics device comprising telescopic denture incorporating a polyaryletherketone**

(30) Priority: 09.01.2014 GB 201400371; 23.01.2014 GB 201401135
(71) Applicant: Juvora Limited, Cleveleys, Lancashire FY5 4QD (GB)
(72) Inventor: Jarman-smith, Marcus, Lytham St. Annes, Lancashire FY8 2EA (GB); Sereno, Nuno, Southport, Merseyside PR8 5BZ (GB); Lobenhofer, Reinhard, Nuernberg (DE)
(74) Representative: Appleyard Lees

(57) **Abstract**

Two stubs (not shown) extend from lower jaw 2 and are capped with primary crowns 4, 6 which are arranged to be releasably engaged with respective secondary crowns 8, 10 provided in a telescopic denture. The primary crowns 4, 6 and the denture 12, including the secondary crowns 8, 10 are made from polyetheretherketone. Methods of making the telescopic denture 12 and other components of the apparatus are also described.

## Description

This invention relates to a prosthodontics device and particularly, although not exclusively, relates to such a device which comprises a telescopic denture.

Prosthodontics devices are arranged to address missing or deficient teeth in patients. One type of known device is referred to as a telescopic denture or German Crown. A telescopic denture comprises a double crown system. A patient's remaining natural teeth are provided with permanent primary crowns made from metal or ceramics. The primary crowns define circular cross-section male projections and are arranged to releasably engage corresponding circular cross-section female elements (e.g. sockets), referred to as secondary crowns, defined in a superstructure which carries prosthetic teeth and gums.

The metal or ceramic primary crowns are time-consuming and expensive to manufacture and must be made with great precision. The same applies to the superstructure comprising the female elements or secondary crowns. For example, primary crowns may be made from hard ceramics by machining; however it can be challenging to machine hard ceramics. It is also known to use softer ceramics which are cured to harden them. However this is a more complex approach which introduces an additional manufacturing step. Furthermore, metal crowns may be made by casting which may produce a less smooth surface with significant surface imperfections. A less smooth surface may have a negative effect in reducing the frictional force between the primary and secondary crowns in use.

Additionally, both ceramics and metal have little flexibility or "give" meaning that the engagement between primary and secondary crowns is relatively rigid which may affect mouth feel. Furthermore, the lack of flexibility during engagement of primary and secondary crowns may mean that the pull off force between the primary and secondary crowns in use is less than optimum.

It is an object of first embodiments of the present invention to address the above identified problems.

Secondary crowns as described are primarily retained on the primary crowns by friction between the male and female elements. However, over a period of time the male and/or female elements become smoother and, consequently, the friction between the primary and secondary crowns is reduced. Eventually the friction is so low that the secondary crown cannot satisfactorily be retained on the primary crown. As a result, the useful lifetime of the secondary crown comes to an end and is discarded. A new superstructure incorporating secondary crowns is, therefore, made for cooperation with the existing primary crowns. However, it is costly and time-consuming to make such a superstructure. It is an object of second embodiments described herein to address the aforementioned problem.

According to a first aspect of the invention, there is provided a prosthodontics device comprising:
a primary crown defining a male element for cooperation with a female element defined by a secondary crown, wherein said primary crown comprises a first polymeric material which comprises a repeat unit of formula (I) 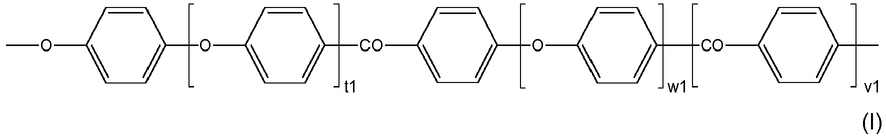 where t1 and w1 independently represent 0 or 1 and v1 represents 0, 1 or 2; and
   a superstructure which includes a secondary crown comprising a female element arranged to cooperate with the primary crown.

In view of the material and/or manufacturing technique (described hereinafter) of the primary crown, the crown may be made thinner than known crowns, meaning that less tooth tissue may need to be removed from a tooth to be provided with a primary crown than hitherto.

Said first polymeric material preferably consists essentially of a repeat unit of formula I. Preferred first polymeric materials comprise (especially consist essentially of) a said repeat unit wherein t1=1, v1=0 and w1=0; t1=0, v1=0 and w1=0; t1=0, w1=1 and v1=2; or t1=0, v1=1 and w1=0. More preferred comprise (especially consist essentially of) a said repeat unit wherein t1=1, v1=0 and w1=0; or t1=0, v1=0 and w1=0. The most preferred comprises (especially consists essentially of) a said repeat unit wherein t1=1, v1=0 and w1=0.

In preferred embodiments, said first polymeric material is selected from polyetheretherketone, polyetherketone, polyetherketoneetherketoneketone and polyetherketoneketone. In a more preferred embodiment, said first polymeric material is selected from polyetherketone and polyetheretherketone. In an especially preferred embodiment, said first polymeric material is polyetheretherketone.

Said first polymeric material may have a Notched Izod Impact Strength (specimen 80mm x 10mm x 4mm with a cut 0.25mm notch (Type A), tested at 23°C, in accordance with ISO180) of at least 4KJm⁻², preferably at least 5KJm⁻², more preferably at least 6KJm⁻². Said Notched Izod Impact Strength, measured as aforesaid, may be less than 10KJm⁻², suitably less than 8KJm⁻². The Notched Izod Impact Strength, measured as aforesaid, may be at least 3KJm⁻², suitably at least 4KJm⁻², preferably at least 5KJm⁻². Said impact strength may be less than 50 KJm⁻², suitably less than 30KJm⁻².

Said first polymeric material suitably has a melt viscosity (MV) of at least 0.06 kNsm⁻², preferably has a MV of at least 0.09 kNsm⁻², more preferably at least 0.12 kNsm⁻², especially at least 0.15 kNsm⁻². Advantageously, the MV may be at least 0.35 kNsm⁻² and especially at least 0.40 kNsm^{-2.} An MV of 0.45 kNsm⁻² has been found to be particularly advantageous in the manufacture of accurate, strong frameworks.

MV is suitably measured using capillary rheometry operating at 400°C at a shear rate of 1000s⁻¹ using a tungsten carbide die, 0.5mmx3.175mm.

Said first polymeric material may have a MV of less than 1.00 kNsm⁻², preferably less than 0.5 kNsm⁻².

Said first polymeric material may have a MV in the range 0.09 to 0.5 kNsm⁻², preferably in the range 0.14 to 0.5 kNsm⁻², more preferably in the range 0.4 to 0.5 kNsm⁻².

Said first polymeric material may have a tensile strength, measured in accordance with ISO527 (specimen type 1b) tested at 23°C at a rate of 50mm/minute of at least 20 MPa, preferably at least 60 MPa, more preferably at least 80 MPa. The tensile strength is preferably in the range 80-110 MPa, more preferably in the range 80-100 MPa.

Said first polymeric material may have a flexural strength, measured in accordance with ISO178 (80mm x 10mm x 4mm specimen, tested in three-point-bend at 23°C at a rate of 2mm/minute) of at least 50 MPa, preferably at least 100 MPa, more preferably at least 145 MPa. The flexural strength is preferably in the range 145-180MPa, more preferably in the range 145-164 MPa.

Said first polymeric material may have a flexural modulus, measured in accordance with ISO178 (80mm x 10mm x 4mm specimen, tested in three-point-bend at 23°C at a rate of 2mm/minute) of at least 1 GPa, suitably at least 2 GPa, preferably at least 3 GPa, more preferably at least 3.5 GPa. The flexural modulus is preferably in the range 3.5-4.5 GPa, more preferably in the range 3.5-4.1 GPa.

Said first polymeric material may be amorphous or semi-crystalline. It is preferably crystallisable. It is preferably semi-crystalline.

The level and extent of crystallinity in a polymer may be measured by wide angle X-ray diffraction (also referred to as Wide Angle X-ray Scattering or WAXS), for example as described by Blundell and Osborn (Polymer 24, 953, 1983). Alternatively, crystallinity may be assessed by Differential Scanning Calorimetry (DSC).

The level of crystallinity of said first polymeric material (suitably measured by DSC) may be at least 1%, suitably at least 3%, preferably at least 5% and more preferably at least 10%. In especially preferred embodiments, the crystallinity may be greater than 25%. It may be less than 50% or less than 40%.

The main peak of the melting endotherm (Tm) of said first polymeric material may be at least 300°C.

Said primary crown may comprise (preferably consist essentially of) a first composition which comprises said first polymeric material. Said first composition suitably includes at least 90 wt%, preferably at least 95 wt%, more preferably at least 99 wt% of said first polymeric material (especially polyetheretherketone). The balance of the first composition may include one or more fillers, for example colourants such as pigments. Preferred fillers may include a material surface that lends itself to polishing. The fillers referred to may be up to around 30 microns diameter. Said first composition suitably includes less than 5 wt%, preferably less than 1 wt%, more preferably 0 wt% hydroxyapatite. Said first composition preferably includes substantially no metal.

Said primary crown preferably includes a region wherein the sides of the crown are parallel to one another. Said region preferably extends a linear distance of at least 2mm, at least 3mm or at least 5mm, suitably measured in a direction parallel to the direction in which the secondary crown is engaged with the primary crown.

Said secondary crown is preferably arranged to engage (e.g. releasably engage) the primary crown by movement of the secondary crown in the direction of a first axis of the primary crown. Said primary crown suitably has a cross-section defined perpendicular to the first axis. Said cross-section of said primary crown is preferably substantially constant (e.g. constant shape and area) along a distance, measured in the direction of the first axis, of at least 2mm, preferably at least 3mm, more preferably at least 4mm, especially at least 5mm. Thus, suitably, in the region of constant cross-section, the sides of the primary crown are parallel to one another. The ratio of the length, measured in the direction of said first axis, of the region of constant cross-section divided by the total length of said first axis (i.e. measured between opposing ends of the primary crown, along the first axis) is suitably at least 0.5, preferably at least 0.6, more preferably at least 0.7, especially at least 0.8. Thus, it will be appreciated that it is preferred for the primary crown to have parallel sides along the majority of its extent.

Said region of constant cross-section may have an area of at least 4mm², preferably at least 5mm², for example at least 7mm² or at least 10mm².

Said region of constant cross-section preferably includes a perimeter which is curved along its entire extent.

Said region of constant cross-section is preferably not of circular cross-section. It may define an oval or other shaped cross-section.

Said primary crown may include a dome-shaped free end, to facilitate initial engagement with the secondary crown in use.

Said secondary crown may comprise a metal (e.g. titanium, cobalt chrome or zirconia), a ceramic material or a thermoplastics material.

Said secondary crown preferably comprises a thermoplastics material. Said secondary crown preferably comprises a second polymeric material which comprises a repeat unit of formula (I) 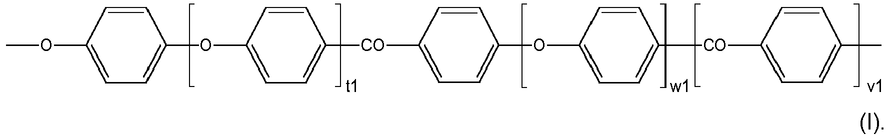 where t1 and w1 independently represent 0 or 1 and v1 represents 0, 1 or 2.

Said second polymeric material may have any feature of the first polymeric material described. Preferably, said first and second polymeric materials are the same. Preferably, they both comprise (preferably consist essentially of) the same repeat unit of formula I, wherein preferably t1=1, w1=0 and v1=0. Said first and second polymeric materials are preferably polyetheretherketone.

Said secondary crown may comprise (preferably consist essentially of) a second composition which comprises said second polymeric material. Said second composition suitably includes at least 90 wt%, preferably at least 95 wt%, more preferably at least 99 wt% of said second polymeric material (especially polyetheretherketone).

Said secondary crown is preferably arranged to releasably engage (e.g. slideably engage) the primary crown.

Said secondary crown preferably incudes a wall which defines an opening of the female element which engages the male element of the primary crown. The cross-section of the opening is preferably substantially constant along a linear distance of at least 2mm, preferably at least 3mm, more preferably at least 4mm, especially at least 5mm.

Said region of constant cross-section may have an area of at least 4mm², preferably at least 5mm², for example at least 7mm² or at least 10mm².

Said region of constant cross-section preferably includes a perimeter which is curved along its entire extent.

Said region of constant cross-section is preferably not of circular cross-section.

Said secondary crown preferably includes a mouth allowing access into the secondary crown, wherein the cross-sectional area of the mouth is at least as large as the cross-sectional area of the opening of the female element inwards of said mouth.

Said superstructure preferably includes at least two secondary crowns which are suitably spaced apart, for example so that, in use, they occupy positions on opposite sides of a patient's mouth. Said two secondary crowns preferably both comprise said second polymeric material and/or said second composition. Said two secondary crowns preferably have the same composition.

Said superstructure preferably includes a joining part which extends between the two secondary crowns, wherein said joining part comprises said second polymeric material. Said joining part preferably comprises the same second polymeric material and/or second composition as said first and second crowns. Said two secondary crowns and said joining part preferably have the same composition and preferably comprise polyetheretherketone. They may be formed by machining from a block which comprises said second polymeric material and/or said second composition.

In one embodiment, said superstructure may include at least three secondary crowns, wherein said superstructure includes joining parts which extend between the secondary crowns, wherein said at least three secondary crowns and said joining parts have the same composition and preferably comprise polyetheretherketone.

Said superstructure preferably includes prosthetic teeth and/or gums. They may be made from acrylic or other plastics material. Said prosthetic teeth and/or gums are preferably secured to a roughened area of the superstructure which has a Ra from 0.06 to 7.0 micrometres, where Ra is the roughness average of a surface, expressed in micrometers. This is the arithmetic mean of the absolute departures of a roughness profile from the mean line of a measurement carried out along an arbitrary line along a surface. Ra is suitably measured by a mechanical contact method by means of a stylus drawn in a straight line over the surface with the height of the stylus measured at regular intervals along the surface, wherein the stylus tip has a diameter of 2 micrometres in accordance with DIN EN ISO 4288 and DIN EN ISO 3274, using a Perthometer SP6 surface profilometer. More preferably, the roughened area may have a Ra from 0.5 to 2.0 micrometres.

Said superstructure suitably includes at least 20 wt% (and preferably less than 35% or less than 30%) of said second polymeric material, preferably a single type of second polymeric material, preferably where t1=1, w1=0 and v1= 0 (especially polyetheretherketone). The balance of said superstructure may be made up of prosthetic teeth and gums. Said superstructure preferably includes substantially no metal.

Said superstructure preferably includes the same number of secondary crowns as there are primary crowns and, preferably, said number is at least two.

Said prosthodontics device preferably includes a female element defined by said secondary crown engaged with the male element defined by said primary crown. Said female element preferably telescopically engages said male element. Each female element defined by secondary crowns preferably telescopically engages a respective primary crown.

According to a second aspect of the invention, there is provided a method of making a prosthodontics device which includes the steps of:
(i) selecting a blank from which a primary crown can be machined, wherein said blank comprises a polymeric material which comprises a repeat unit of formula (I) 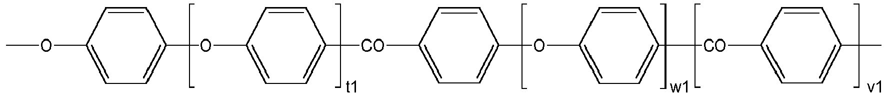 where t1 and w1 independently represent 0 or 1 and v1 represents 0, 1 or 2;
(ii) using digital technology to collate data to define the shape and/or dimensions of the primary crown; and;
(iii) machining the blank in dependence upon the data.

Said prosthodontics device and/or primary crown may have any feature of the prosthodontics device and/or primary crown of the first aspect. Said blank preferably comprises a first polymeric material or a first composition as described according to the first aspect.

In step (ii), digital technology is used to collate data on the region onto which the primary crown is to fit. Step (ii) preferably includes scanning a region into which the primary crown is to fit (e.g. scanning a patient's mouth) or scanning of a model of a region into which the primary crown is to fit. Preferably, data is collated from a model, for example a cast, obtained of a patient's mouth and/or dentition. Step (ii) may comprise use of Computer-aided design (CAD) technology.

The method may include a step prior to step (ii) of taking an impression of a patient's mouth. The impression may be used to collate said data.

The method preferably involves a CAD/CAM technique whereby data is collated as aforesaid and computer-aided manufacture (CAM) is undertaken in step (iii). Thus, in step (iii), a computer suitably controls the machining of the blank.

Preferably, the selected blank is positioned in a CAD/CAM machine and the machine is arranged to machine the blank in dependence upon the data.

Preferably, machining of said blank is undertaken using at least 5-axis machining, suitably under computer control. In some cases, higher axis (e.g. 7-axis) machining may be undertaken. Machining in step (iii) suitably comprises milling. The work piece is suitably cooled during machining so as to control crystallinity of the machined blank.

According to a third aspect of the invention, there is provided a method of making a prosthodontics device which includes the steps of:
(a) selecting a blank from which a secondary crown can be machined, wherein said blank comprises a polymeric material which comprises a repeat unit of formula (I) 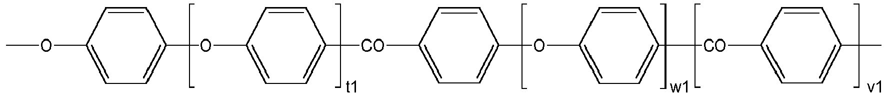 where t1 and w1 independently represent 0 or 1 and v1 represents 0, 1 or 2;
(ii) using digital technology to collate data to define the shape and/or dimensions of the secondary crown; and;
(iii) machining the blank in dependence upon the data.

In step (ii), digital technology is used to collate data on the region onto which the secondary crown is to fit. Step (ii) preferably includes scanning a region into which the secondary crown is to fit (e.g. scanning a patient's mouth) or scanning of a model of a region into which the secondary crown is to fit. Preferably, data is collated from a model, for example a cast, obtained of a patient's mouth and/or dentition. Step (ii) may comprise use of Computer-aided design (CAD) technology.

The method may include a step prior to step (ii) of taking an impression of a patient's mouth. The impression may be used to collate said data.

The method preferably involves a CAD/CAM technique whereby data is collated as aforesaid and computer-aided manufacture (CAM) is undertaken in step (iii). Thus, in step (iii), a computer suitably controls the machining of the blank.

Preferably, the selected blank is positioned in a CAD/CAM machine and the machine is arranged to machine the blank in dependence upon the data.

Preferably, machining of said blank is undertaken using at least 5-axis machining, suitably under computer control. In some cases, higher axis (e.g. 7-axis) machining may be undertaken. Machining in step (iii) suitably comprises milling. The work piece is suitably cooled during machining so as to control crystallinity of the machined blank.

The method of the third aspect may include, in step (ii), using digital technology to collate data to define the shape, position and/or dimensions of at least two secondary crowns (e.g. by determining the shape, position and/or dimensions of at least two primary crowns in a patient's mouth); and then, suitably in step (iii), machining the blank in dependence upon said data. Preferably, the blank is machined to define a structure (e.g. a unitary and/or monolithic structure) which includes all secondary crowns to be defined in the prosthodontics device and one or more joining parts (e.g. as described according to the first aspect) extending between the secondary crowns for securing the secondary crowns in position relative to one another.

The method may include the step, after step (iii), of securing prosthetic teeth and/or gums to the machined blank. Prior to this, areas of the machined blank may be treated to adjust, for example, increase the Ra of said areas. The Ra may be adjusted in said areas so it is as described in the first aspect. Al₂O₃ particles may suitably be used as abrasive particles for blasting areas in order to provide roughening. Alternatively or additionally, sulphuric acid etching or tribochemical treatments may be applied.

Any suitable dental veneer may be used to define the prosthetic teeth and/or gums. However, it has been found that optimal bonding occurs when the dental veneer comprises silica, for instance at least 10% by weight of silica. The dental veneer may be a pre-formed veneer or may be formed in-situ.

An opaque paste comprising silica may be located between the dental veneer and the machined blank. Such opaque pastes are commercially available for use in bonding crowns to cores of tooth prostheses, and are used to provide a realistic tooth-like appearance to the crown. It has been found that the use of an opaque paste comprising silica may improve the bonding of the dental veneer relative to the machined blank.

A bonding layer may be located between an outer face of the machined blank and the dental veneer. The bonding layer may comprise reactive phosphate or silane moieties.

In a preferred embodiment, the method of the second aspect precedes the method of the third aspect. Thus, in a preferred embodiment, the invention extends to the method of the second aspect followed by the method of the third aspect.

According to a fourth aspect of the invention, there is provided a primary crown as described according to the first aspect per se. The primary crown suitably includes a recess which is adapted to engage a tooth stub and any other feature of the primary crown described herein. The primary crown may include machining lines confirming it has been produced by machining and suitably consists essentially of polyetheretherketone.

According to a fifth aspect of the invention, there is provided a superstructure which includes a secondary crown as described according to the first aspect per se. The superstructure suitably includes machining lines confirming it has been produced by machining and suitably includes at least 75 wt% of polyetheretherketone.

According to a sixth aspect, there is provided a method of providing a prosthodontics device (e.g. as described according to the first aspect) comprising:
(i) securing at least two primary crowns to respective tooth stubs, said primary crowns preferably being made as described according to the second aspect;
(ii) releasably securing a superstructure which includes at least two secondary crowns to the primary crowns, said superstructure preferably being made as described according to the third aspect;
wherein preferably said superstructure is slideably engaged with said primary crown and preferably no cement is used to secure said superstructure in position.

According to a seventh aspect of the invention, there is provided a prosthodontics device comprising a superstructure which includes a secondary crown comprising a female element arranged to cooperate with a primary crown, wherein said female element is provided with a lining which comprises a first polymeric material which comprises a repeat unit of formula (I) 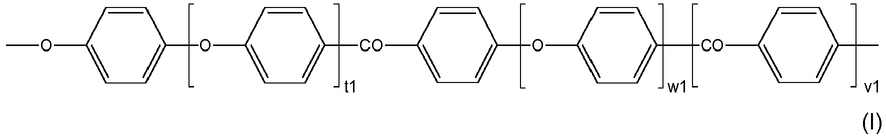 where t1 and w1 independently represent 0 or 1 and v1 represents 0, 1 or 2.

Said first polymeric material of the seventh aspect may have any feature of the first polymeric material of the first aspect.

Said lining may comprise (preferably consist essentially of) a first material which comprises said first polymeric material. Said first material suitably includes at least 90 wt%, preferably at least 95 wt%, more preferably at least 99 wt% of said first polymeric material (especially polyetheretherketone). The balance of the first material may include one or more fillers. Said first material suitably includes less than 5 wt%, preferably less than 1 wt%, more preferably 0 wt% hydroxyapatite. Said first material preferably includes substantially no metal.

Said lining of said secondary crown is preferably arranged to cooperate with the primary crown, suitably so a wall of the secondary crown makes face to face contact with a wall of the primary crown suitably so there is substantially no gap between the primary crown and a surface of the secondary crown which overlies the primary crown. Other features of the primary and secondary crowns are described with reference to the assembly of the eighth aspect.

Said secondary crown is preferably arranged to releasably engage (e.g. slideably engage) the primary crown.

Said secondary crown preferably incudes a wall which defines an opening of the female element which is provided with said lining and is arranged to engage the male element of the primary crown. The cross-section of the opening is preferably substantially constant along a linear distance of at least 2mm, preferably at least 3mm, more preferably at least 4mm, especially at least 5mm. Said region of constant cross-section may have an area of at least 4mm², preferably at least 5mm², for example at least 7mm² or at least 10mm². Said region of constant cross-section preferably includes a perimeter which is curved along its entire extent. Said region of constant cross-section is preferably not of circular cross-section.

Said secondary crown preferably includes a mouth allowing access into the secondary crown, wherein the cross-sectional area of the mouth is at least as large as the cross-sectional area of the secondary crown inwards of said mouth.

The female element of the secondary crown may be made of any material, for example any material used to make secondary crowns. For example, it may be made from a metal (e.g. CoCr or titanium), a ceramic (e.g. zirconia) or a plastics material, for example a polymeric material, for example a thermoplastic polymeric material such as said first polymeric material described. Thus, a female element of any material may be provided with a lining comprising said first polymeric material as described.

Said superstructure preferably includes at least two secondary crowns which are suitably spaced apart, for example so that, in use, they occupy positions on opposite sides of a patient's mouth. Said two secondary crowns preferably both include respective female elements, wherein both female elements are provided with a respective lining which comprises said first polymeric material and/or comprises said first material. The linings of both female elements preferably comprise the same material.

When said superstructure includes at least two secondary crowns, said superstructure preferably includes a joining part which extends between the two secondary crowns. Said superstructure may be made of any material, for example any material used in making superstructures of secondary crowns. For example, it may be made from a metal (e.g. CoCr or titanium), a ceramic (e.g. zirconia) or a plastics material, for example a polymeric material, for example a thermoplastic polymeric material such as said first polymeric material described. Said joining part preferably comprises the same material as said first and second crowns. Said two secondary crowns and said joining part preferably have the same composition.

In one embodiment, said superstructure may include at least three secondary crowns, wherein said superstructure includes joining parts which extend between the secondary crowns, wherein said at least three secondary crowns and said joining parts have the same composition. Each of said three secondary crowns suitably includes a respective female element which is preferably provided with a lining which comprises said first polymeric material and/or said first material. The linings preferably each have the same composition.

Said superstructure may include prosthetic teeth and/or gums which may be as described for the prosthetic teeth and/or gums of the first aspect.

Said lining suitably has a thickness of less than 100µm, preferably less than 75µm, more preferably less than 50µm. The thickness may be at least 6µm. Preferably, said thickness is in the range 6 to 100µm, preferably 6 to 30µm. Said lining may have a thickness as aforementioned across at least 50% (preferably at least 80%, especially about 100%) of its surface area. Said lining preferably has a substantially constant thickness across substantially its entire extent.

Said lining preferably covers at least 80%, more preferably about 100% of the area of a surface which defines the female element.

A surface of the lining which contacts the female element is preferably roughened. Said surface which contacts preferably has a Ra from 0.06 to 7.0µm where Ra is as described above. Preferably, said surface has a Ra from 0.5 to 2.0µm.

Said lining may be secured within the female element by way of an adhesive; or, alternatively, it may be secured by welding (e.g. where both the female element and lining are defined by a thermoplastic, for example by a said first polymeric material).

Said superstructure preferably defines a telescopic denture.

According to an eighth aspect of the invention, there is provided an assembly comprising:
(i) a prosthodontics device according to the seventh aspect;
(ii) a primary crown defining a male element which cooperates with the secondary crown of said prosthodontics device.

Said primary crown of the assembly of the eighth aspect may be as described for the primary crown of the first aspect.

Said second crown of the assembly of the eighth aspect may be as described for the secondary crown of the first aspect.

Said primary crown preferably comprises a thermoplastics material. Said primary crown preferably comprises a second polymeric material which comprises a repeat unit of formula (I) 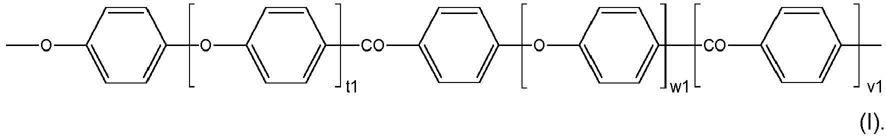 where t1 and w1 independently represent 0 or 1 and v1 represents 0, 1 or 2.

Said second polymeric material of said primary crown of the eighth aspect may have any feature of the first polymeric material described according to the seventh aspect. Preferably, said first polymeric material of the lining of the seventh aspect and the second polymeric material of the primary crown of the eighth aspect are the same. Preferably, they both comprise (preferably consist essentially of) the same repeat unit of formula I, wherein preferably t1=1, w1=0 and v1=0. Said first and second polymeric materials are preferably polyetheretherketone.

Said primary crown may comprise (preferably consist essentially of) a second material which comprises said second polymeric material. Said second material suitably includes at least 90 wt%, preferably at least 95 wt%, more preferably at least 99 wt% of said second polymeric material (especially polyetheretherketone).

According to a ninth aspect of the invention, there is provided a method of making a lining for a female element of a secondary crown (e.g. a telescopic denture), the method comprising:
(a) selecting a precursor which comprises a first polymeric material as described according to the seventh aspect; and
(b) shaping the precursor to define said lining.

Said lining may have any feature of the lining of the seventh aspect.

The method preferably comprises assessing the shape of the female element to be lined by said lining. Assessing the shape may comprise preparing a mould of an appropriate region of said female element or digital technology may be used to collate data which describes the shape of an appropriate region of said female element. In a first embodiment, which involves preparing a mould, the mould may be used in thermoforming a film which comprises said first polymeric material in order to define said lining. In this case, a mould may also be prepared of a primary crown with which the secondary crown is to cooperate in use. A film is then thermoformed between the two moulds.

In a second embodiment which involves preparing a mould, the mould may be used to collate data which defines the shape of the female element and the data may then be used in a CAD/CAM technique to prepare (e.g. mill) the lining from the precursor which may be in the form of a blank (e.g. disc or block) which comprises said first polymeric material.

As an alternative to taking a mould, digital technology may be used to define the shape of the lining by scanning the shape of the female element to be lined by said lining. A CAD-CAM technique may then be used to prepare (e.g. mill) the insert from the precursor which may be in the form of a blank (e.g. disc or block) which comprises said first polymeric material.

According to a tenth aspect of the invention, there is provided a collocation comprising a lining for a female element of a secondary crown in combination with information relating to the shape of a female element in which the lining may be fitted or in combination with information relating to a patient having a primary crown with which the female element may cooperate.

The collocation may include said lining provided in a package, for example a receptacle, in combination with information sufficient to identify a patient having a primary crown with which the female element may cooperate. Said lining may have any feature of the lining of the seventh aspect.

According to an eleventh aspect of the invention, there is provided a method of refurbishing a prosthodontics device (e.g. a telescopic denture) which includes a secondary crown comprising a female element arranged to cooperate with a primary crown, the method comprising:
(i) selecting a lining which comprises a first polymeric material which comprises a repeat unit of formula (I) 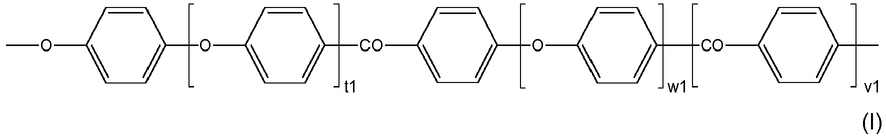 where t1 and w1 independently represent 0 or 1 and v1 represents 0, 1 or 2; and
(ii) securing the lining within the female element thereby to line the female element.

The lining may be made as described according to the seventh aspect. The lining may be as described according to the ninth aspect.

After the lining has been secured as described in step (ii), a prosthodontics device as described in the seventh aspect may be defined.

In the method, the lining may be secured in step (ii) using an adhesive means; or, in some cases, it may be treated with heat (e.g. as in ultrasonic welding) to secure it in position. Said lining is preferably substantially permanently secured in position in the method.

The lining selected in step (i) may include a wall having a thickness of less than 100µm, for example in the range 6 to 100µm.

The lining may have a Ra as described herein.

Any invention described herein may be combined with any feature of any other invention described herein mutatis mutandis.

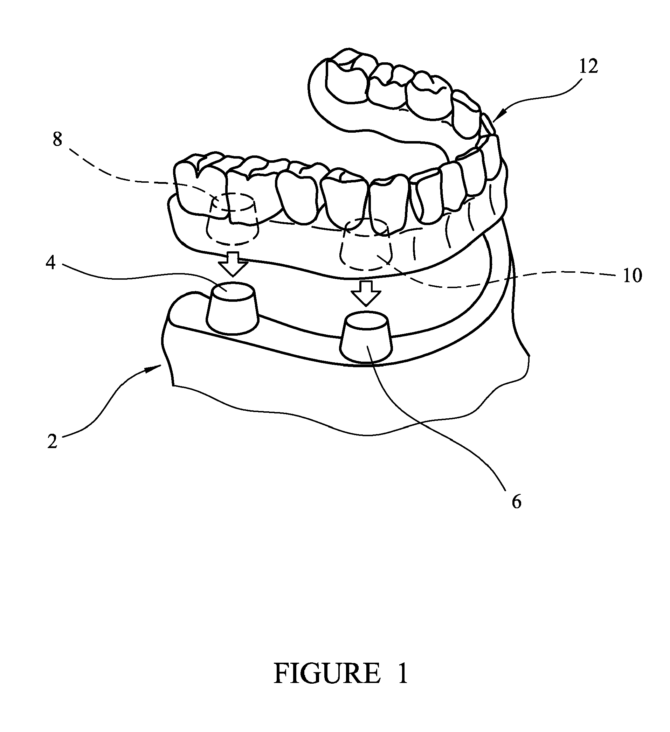

Specific embodiments of the invention will now be described, by way of example, with reference to Figure 1 which is a front perspective view of parts of a telescopic denture.

Referring to the figure, tooth stubs (not shown) extend from lower jaw 2 and are capped with primary crowns 4, 6 which are arranged to be releasably engaged with respective secondary crowns 8, 10 provided in a telescopic denture 12. It will be appreciated that, suitably, there are additional tooth stubs capped with primary crowns spaced from those shown in the figure. Advantageously, the primary crowns 4, 6 (including any provided but not shown) and the denture 12, including the secondary crowns 8, 10 (including any provided but not shown), are made from polyetheretherketone (PEEK). This material is available from Invibio Limited under the Trade Mark PEEK-OPTIMA. Grade LT1, LT2 or LT3 may be selected. Further detail is provided below.

Firstly, a patient's existing teeth are prepared, for example by being formed into stubs which can be capped with crowns. Then a mould is taken of the patient's mouth using a standard impression tray. The mould is poured with dental plaster and allowed to set. The mould is next scanned to collate relevant CAD data which is input into a 5 axis CAD-CAM machine, followed by the design of the primary crown using CAD software tools. The machine then produces the primary crowns from a PEEK disc. The internal surfaces of the primary crowns are then roughened using a grit blasting procedure and are then secured onto the stubs using a dental cement.

Next, a second mould is taken of the patient's mouth using a standard impression tray and the mould poured in the dental tray which is allowed to set. This mould is then scanned to collate relevant CAD data which is input into the 5 axis CAD-CAM machine, followed by the design of the denture using CAD software tools. The machine then produces the telescope denture 12 including the secondary crowns 8,10 (which comprise female elements).

The denture produced may be finalised by application of dental veneers to define aesthetically- acceptable prosthetic teeth and gums.

The telescopic denture 12 may be releasably secured in the mouth by engagement of respective secondary crowns 8, 10 which define female elements with primary crowns 4, 6 which define male elements. The denture 12 is releasably secured in position by friction between the primary and secondary crowns. Typically pull off force between denture 12 and primary crown may be 10 to 20 N. This may be tested in a Universal Testing Machine (UTM Zwick 1446) using tensile test design.

The arrangement described may be advantageous for the following reasons:
1. Because of the accuracy of manufacture of the primary and secondary crowns, the pull off force may be relatively high.
2. The primary crowns may display a better fit, on the stubs than conventional cast metal alternatives.
3. Since the primary and secondary crowns are made from PEEK, this may deliver a beneficial and more forgiving fit on placement of the telescope denture onto the PEEK primary crowns.
4. The telescope denture incorporating a PEEK secondary crown, will slide in a more controllable and comfortable way in relation to the primary crowns, as less force is required to be applied by the patient. This is due to the relatively low coefficient of friction of the PEEK (0.4) when compared with other materials such as ceramic or zirconium.
5. In view of the higher dimensional accuracy involved in preparation of the primary crown, compared, for example to cast metals, the gap between the primary and secondary PEEK crowns can be smaller than in the cast crowns. This smaller gap may mean that, once in place, improved retention of the telescope denture is observed.
6. Use of PEEK as described may provide cheaper alternatives to options such as gold or certain ceramic crowns.

When the frictional force between the primary crowns 4, 6 and secondary crowns 8, 10 is insufficient to retain the telescopic denture 12 on the primary crowns 4, 6, the secondary crowns may be re-lined with a thin layer of polyetheretherketone (PEEK) so as to increase the frictional force between the primary and secondary crowns and thereby extend the useful lifetime of the telescopic denture 12.

In embodiment (I), a PEEK film may be used to re-line a secondary crown which is made from metal as follows:
A (i) A PEEK film of thickness in the range 6-100µm, preferably 6 to 30 µm, is selected and thermoformed to match and fit snugly within a socket defined by the secondary crown.
   A first mould half for thermoforming is taken of the socket of the secondary crown and a second mould half is taken of the primary crown. The mould halves are made from a material which can withstand temperatures higher than the temperature required to thermoform PEEK. Then, the selected PEEK film is encased between the mould halves and the combination is heated, under pressure, above the glass transition temperature (Tg) of the PEEK. The excess film is then removed leaving a thermoformed insert which has an external surface which corresponds to that of the socket of the secondary crown.
A (ii) The external surface of the thermoformed insert is then treated to facilitate its bonding within the socket of the secondary crown. This may comprise:
   (a) grit blasting the external surface to roughen the surface. This may involve use of 50µm aluminium oxide particles at a pressure of 2 bar.
   (b) plasma treatment (e.g. using argon gas, power 50W, pressure 0.4mb, for 15 minutes) or laser surface treatment (e.g. 355nm, 70mJ, 20mm/mn) of the roughed surface to enhance the free energy of the surface and improve fixation to subsequently applied bonding agents.
A (iii) The surface which defines the socket of the secondary crown is treated, for example by grit blasting or acid etching, to improve its ability to bond to the thermoformed insert.
A (iv) The thermoformed insert is then adhesively secured within the socket. For example, a primer (e.g. a non-curing silicone primer SP-270, NuSil Technology) may be applied to the outer surface of the thermoformed insert. Then an adhesive (e.g. implantable silicone adhesive MED-1511, NuSil Technology) is applied to the outer surface. The insert is then pushed into the socket and cured under applied pressure at ambient temperature.

In Embodiment (II), a PEEK film may be used to re-line a secondary crown which is made from PEEK. In this case, the PEEK film and steps taken may generally be as described in Embodiment (I) except for the following:
B (i) The surface which defines the socket of the secondary crown (which in this case is made from PEEK) is roughed as described in Embodiment (I) and, additionally, is plasma treated or laser treated as described in A (i) above to enhance its free energy.
B (ii) The thermoformed insert may be secured in position as described in A (iv) or it may be secured by being ultrasonically welded in position using a hand-held sonotrode having ultrasonic power supplied thereto (70 KHZ at 100W). To achieve a good weld, it is preferred to use high vibration amplitudes (e.g. 70µm), a weld pressure of 5 MPa and a weld time of about 1 second.

In Embodiment (III), as an alternative to use of a PEEK film to produce a thermoformed insert, an insert for the socket of the secondary crown may be made by milling. For example, a mould may be taken of the socket of the secondary crown using a moulding material or information on the socket may be collated by digitally scanning the socket. Where a moulding material is used, the mould may be scanned to collate relevant digital information. The collated information may then be input into a 5-axis CAD-CAM machined, followed by design of the insert. The CAD-CAM machine may then be used to produce the insert from a block or disc of PEEK. The insert may then be secured within the socket as described for Embodiments (I) and (II).

Thus, using the procedures of Embodiments (I), (II) and (III), inserts made from PEEK may readily be produced and secured in position in sockets of secondary crowns, thereby relining the secondary crowns and prolonging the useful lifetime of telescopic dentures in a cost effective manner.

The invention is not restricted to the details of the foregoing embodiment(s). The invention extends to any novel one, or any novel combination, of the features disclosed in this specification (including any accompanying claims, abstract and drawings), or to any novel one, or any novel combination, of the steps of any method or process so disclosed.

## Claims

1. A prosthodontics device comprising:
a primary crown defining a male element for cooperation with a female element defined by a secondary crown, wherein said primary crown comprises a first polymeric material which comprises a repeat unit of formula (I) 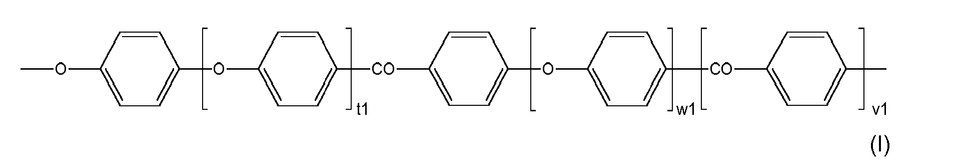
where t1 and w1 independently represent 0 or 1 and v1 represents 0, 1 or 2; and
a superstructure which includes a secondary crown comprising a female element arranged to cooperate with the primary crown.

2. A device according to claim 1, wherein said first polymeric material comprises (especially consists essentially of) a said repeat unit wherein t1=1, v1=0 and w1=0; and, preferably, said polymeric material has an MV in the range 0.09 to 0.5 kNsm⁻²

3. A device according to any preceding claim, wherein said primary crown comprises a first composition which comprises said first polymeric material, wherein said first composition includes at least 90wt%, preferably at least 99wt%, of said first polymeric material; and, preferably, said first composition includes substantially no metal.

4. A device according to any preceding claim, wherein said primary crown includes a region wherein the sides of the crown are parallel to one another and said region extends a linear distance of at least 2mm; and/or wherein said secondary crown is arranged to engage the primary crown by movement of the secondary crown in the direction of a first axis of the primary crown, wherein said primary crown has a cross-section defined perpendicular to the first axis which is substantially constant along a distance, measured in the direction of the first axis, of at least 2mm, preferably at least 5mm; wherein, optionally, said region of substantially cross-section has an area of at least 4mm² and/or said region of substantially constant cross-section includes a perimeter which is curved along its entire extent.

5. A device according to any preceding claim, wherein said secondary crown comprises a second polymeric material which comprises a repeat unit of formula 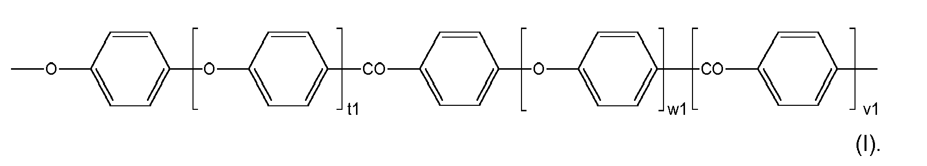 where t1 and w1 independently represent 0 or 1 and v1 represents 0, 1 or 2.

6. A device according to claim 5, wherein said secondary crown comprises a second composition which comprises said second polymeric material, wherein said second composition includes at least 90wt%, preferably at least 99wt% of said second polymeric material.

7. A device according to any preceding claim, wherein said secondary crown is arranged to releasably engage the primary crown; and said secondary crown includes a wall which defines an opening of the female element which engages the male element of the primary crown, wherein the cross-section of the opening is substantially constant along a linear distance of at least 2mm, preferably at least 5mm, and/or said region of constant cross section has an area of at least 4mm²; and wherein, preferably, said region of constant cross-section includes a perimeter which is curved along its entire extent.

8. A device according to any preceding claim, wherein said superstructure includes at least two secondary crowns which are spaced apart so that, in use, they occupy positions on opposite sides of a patient's mouth.

9. A device according to claim 8, wherein said superstructure includes a joining part which extends between the two secondary crowns, wherein said joining part comprises said second polymeric material.

10. A device according to any preceding claim, wherein said superstructure includes prosthetic teeth and/or gums; and/or wherein said superstructure includes no metal.

11. A device according to claim 10, wherein said prosthetic teeth and/or gums is/are secured to a roughened area of the superstructure which has a Ra from 0.06 to 7.0 micrometers, wherein Ra is the roughness average of a surface, expressed in micrometers.

12. A method of making a prosthodontics device which includes the steps of:
(A)
(i) selecting a blank from which a primary crown can be machined, wherein said blank comprises a polymeric material which comprises a repeat unit of formula (I) 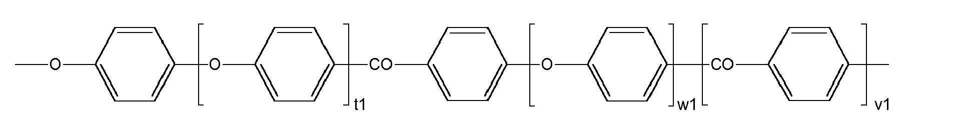 where t1 and w1 independently represent 0 or 1 and v1 represents 0, 1 or 2;
(ii) using digital technology to collate data to define the shape and/or dimensions of the primary crown; and
(iii) machining the blank in dependence upon the data; and/or the steps of:
(B)
(i) selecting a blank from which a secondary crown can be machined, wherein said blank comprises a polymeric material which comprises a repeat unit of formula (I) 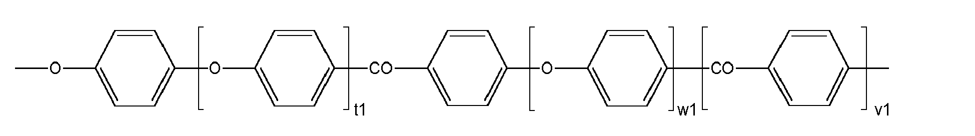 where t1 and w1 independently represent 0 or 1 and v1 represents 0, 1 or 2;
(ii) using digital technology to collate data to define the shape and/or dimensions of the secondary crown; and
(iii) machining the blank in dependence upon the data.

13. A method according to claim 12, wherein said prosthodontics device and/or primary crown and/or second crown have any feature of the prosthodontics device and/or primary crown and/or secondary crown described in any of claims 1 to 11.

14. A method according to claim 12 or claim 13, wherein the work piece is cooled during machining so as to control crystallinity of the machined blank; and/or step (B) (ii) comprises using digital technology to collate data to define the shape, position and/or dimensions of at least two secondary crowns (e.g. by determining the shape, position and/or dimensions of at least two primary crowns in a patient's mouth); and then, in step (B) (iii), machining the blank in dependence upon said data, wherein the blank is machined to define a structure which includes all secondary crowns to be defined in the prosthodontics device and one or more joining parts extending between the secondary crowns for securing the secondary crowns in position relative to one another.

15. A method according to any of claims 12 to 14, wherein the method comprises treating areas of the blank prior to securing prosthetic teeth and/or gums to the machined blank, to increase the Ra of said areas; and/or wherein prosthetic teeth and/or gums are defined by a dental vaneer which comprises silica.
